(19) Europäisches Patentamt
European Patent Office
Office européen des brevets

(11) Veröffentlichungsnummer: **0 122 407**
**A2**

# (12) EUROPÄISCHE PATENTANMELDUNG

(21) Anmeldenummer: **84101886.4**

(22) Anmeldetag: **23.02.84**

(51) Int. Cl.³: **A 01 N 43/50**
//C07D233/56

(30) Priorität: **05.03.83 DE 3307909**

(43) Veröffentlichungstag der Anmeldung:
**24.10.84 Patentblatt 84/43**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Jäger, Gerhard, Dr.**
**Gellertstrasse 18**
**D-5090 Leverkusen 1(DE)**

(72) Erfinder: **Reinecke, Paul, Dr.**
**Lessingstrasse 11**
**D-5090 Leverkusen 3(DE)**

(54) **3,3-Diphenyl-3-(imidazol-1-yl)-propin als fungizides Mittel.**

(57) Die Erfindung betrifft die spezielle Verwendung des bekannten 3,3-Diphenyl-3-(imidazol-1-yl)-propins als Fungizid gegen Pseudocercosporella herpotrichoides.

Die nach bekannten Verfahren herstellbare Verbindung der Formel

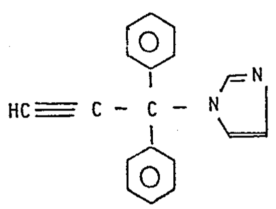

ist als Pflanzenschutzmittel verwendbar.

EP 0 122 407 A2

BEZEICHNUNG GEÄNDERT
siehe Titelseite

BAYER AKTIENGESELLSCHAFT
5090 Leverkusen, Bayerwerk
Konzernverwaltung RP
Patentabteilung
Slr/Hcd
II b

# Fungizides Mittel, Verfahren zu dessen Herstellung, sowie dessen Verwendung

Die vorliegende Erfindung betrifft die spezielle Verwendung des bekannten 3,3-Diphenyl-3-(imidazol-1-yl)-propins als Fungizid gegen Pseudocercosporella herpotrichoides.

Es ist bereits bekannt geworden, daß bestimmte substituierte Azolylpropine, wie auch das 3,3-Dimethyl-3-(imidazol-1-yl)-propin, gute fungizide Wirksamkeit besitzen (vergleiche DE-OS 21 28 700 [LeA 13 716]). Ueber die vom wirtschaftlichen Standpunkt aus hoch interessante Wirksamkeit gegenüber Pseudocercosporella herpotrichoides ist jedoch nichts bekannt.

Es wurde gefunden, daß speziell das 3,3-Diphenyl-3-(imidazol-1-yl)-propin der Formel

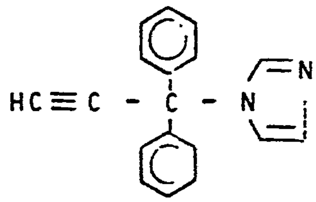

(I)

eine besonders gute fungizide Wirksamkeit gegen Pseudocercosporella herpotrichoides aufweist.

Ueberraschenderweise zeigt das 3,3-Diphenyl-3-(imidazol-1-yl)-propin der Formel (I) eine erheblich bessere fungizide Wirkung als andere aus dem Stand der Technik bekannte substituierte Azolylpropine, wie beispielsweise 3-(Imidazol-1-yl)-3-isopropyl-3-phenyl-propin, welches chemisch eine naheliegende Verbindung ist.
Die neue Verwendung des Wirkstoffes stellt somit eine Bereicherung der Technik dar.

Das erfindungsgemäß zu verwendende 3,3-Dimethyl-3-(imidazol-1-yl)-propin der Formel (I) ist bekannt (vergleiche DE-AS 20 44 621 [LeA 13 283]). Es kann durch Umsetzung von 3,3-Diphenyl-propin-3-ol (vergleiche J. Amer. Chem. Soc. 83, 4990 (1961))mit Thionyl-bis-imidazol hergestellt werden. Besonders vorteilhaft kann es durch Reaktion von 3,3-Diphenyl-propin-3-ol mit Azoliden anderer anorganischer Säuren, wie beispielsweise Carbonyl-bis-imidazol oder Phosphorsäure-tris-imidazolid erhalten werden.

Der erfindungsgemäße Wirkstoff weist eine starke mikrobizide Wirkung auf und kann zur Bekämpfung von unerwünschten Mikroorganismen praktisch eingesetzt werden. Der Wirkstoff ist für den Gebrauch als Pflanzenschutzmittel geeignet.

Die gute Pflanzenverträglichkeit des Wirkstoffes in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel kann der erfindungsgemäße Wirkstoff mit besonders gutem Erfolg zur Bekämpfung von Pseudocercosporella, dem Erreger der Halmbruchkrankheit an Getreide, eingesetzt werden.

Der Wirkstoff kann in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfs lösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Der Wirkstoff kann als solcher in Form seiner Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen kann die Wirkstoffkonzentration in den Anwendungsformen in einem größeren Bereich variiert werden. Sie liegt im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

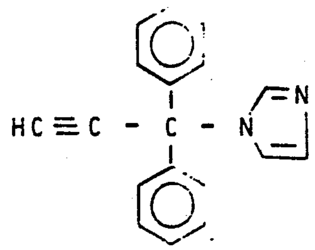

860g (7,5 Mol) Phosphorsäure-tris-imidazolid und 1400g (6,73 Mol) 3,3-Diphenylpropin-3-ol werden in 5 l Acetonitril zusammengegeben und 48 Stunden unter Rückfluß erhitzt. Anschließend werden innerhalb von 3 Stunden 4 l Acetonitril bei Normaldruck abdestilliert und der Rückstand in 15 l Wasser verrührt. Man extrahiert mit 8 l Methylenchlorid, trennt die organische Phase ab und destilliert 7,5 l Methylenchlorid ab. Zu dem Rückstand werden 900 ml Acetonitril gegeben und gekühlt. Das ausgeschiedene Kristallisat wird abgenutscht, mit 1 l kaltem Acetonitril angeschlämmt, trockengesaugt und bei 50°C im Umlüfter getrocknet. Man erhält 750g (43 % der Theorie) 3,3-Diphenyl-3-(imidazol-1-yl)-propin vom Schmelzpunkt 134-136°C.

Verwendungsbeispiele

In dem nachfolgenden Verwendungsbeispiel wird die nachstehend angegebene Substanz als Vergleichsverbindung eingesetzt:

(A) 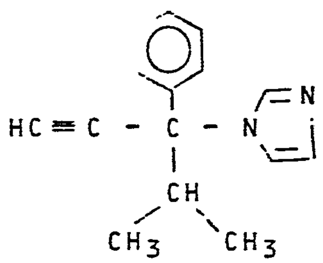

Beispiel A

Pseudocercosporella-Test (Getreide) / Sproßbehandlung/ Feldversuch

Getreideart : Winterweizen
Parzellengröße : 1 $m^2$
Anzahl der Wiederholungen: 3

Die Anwendung der Wirkstoffe erfolgt in handelsüblichen Formulierungen beim Schossen des Getreides.

Die Auswertung wird zu dem Zeitpunkt, bei dem die Krankheitssymptome vollständig und gut zu erkennen sind, durchgeführt.

Eine deutliche Ueberlegenheit in der Wirksamkeit gegenüber dem Stand der Technik zeigt bei diesem Test die Verbindung gemäß Herstellungsbeispiel 1.

Tabelle: A

Pseudocercosporella-Test (Getreide) / Sproßbehandlung/

Feldversuch

| Wirkstoff | Wirkstoffaufwandmenge in g / ha | Krankheitsbefall in % der unbehandelten Kontrolle |
|---|---|---|
| 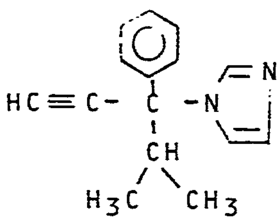<br>(A) (bekannt) | 360 | 100 |
| 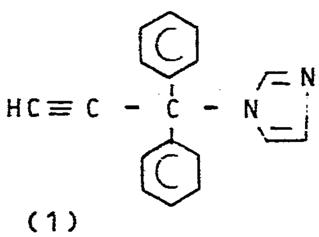<br>(1) | 360 | 19,5 |

Patentansprüche

1. Fungizides Mittel zur Bekämpfung von Pseudocercosporella herpotrichoides, gekennzeichnet durch einen Gehalt an 3,3-Diphenyl-3-(imidazol-1-yl)-propin der Formel

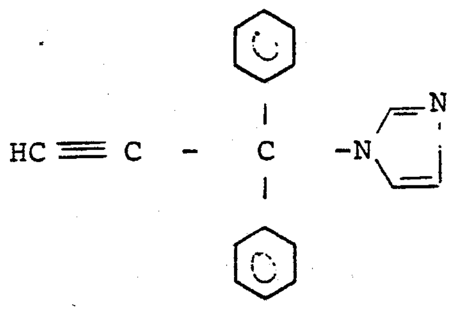

(I)

2. Verfahren zur Bekämpfung von Pseudocercosporella herpotrichoides, dadurch gekennzeichnet, daß man 3,3-Diphenyl-3-(imidazol-1-yl)-propin der Formel (I) in Anspruch 1 auf Pilze dieser Art oder deren Lebensraum einwirken läßt.

3. Verwendung von 3,3-Diphenyl-3-(imidazol-1-yl)-propin der Formel I in Anspruch 1, als Pflanzenschutzmittel zur Bekämpfung von Pseudocercosporella herpotrichoides.

4. Verfahren zur Herstellung eines fungiziden Mittels zur Bekämpfung von Pseudocercosporella herpotrichoides, dadurch gekennzeichnet, daß man 3,3-Diphenyl-3-(imidazol-1-yl)-propin der Formel I in Anspruch 1 mit Streckmitteln und/oder oberflächenaktiven Mitteln vermischt.